(19) Europäisches Patentamt / European Patent Office / Office européen des brevets

(11) **EP 2 379 590 B1**

(12) **EUROPEAN PATENT SPECIFICATION**

(45) Date of publication and mention of the grant of the patent:
**24.01.2018 Bulletin 2018/04**

(21) Application number: **09837212.1**

(22) Date of filing: **31.12.2009**

(51) Int Cl.:
*C07K 16/08* (2006.01)         *A61K 39/395* (2006.01)
*G01N 33/569* (2006.01)

(86) International application number:
**PCT/US2009/069952**

(87) International publication number:
**WO 2010/078518 (08.07.2010 Gazette 2010/27)**

(54) **ANTI-HERPES SIMPLEX VIRUS ANTIBODIES AND METHODS OF USE THEREOF**

ANTIKÖRPER GEGEN HERPES SIMPLEX VIRUS UND VERFAHREN ZU IHRER VERWENDUNG

ANTICORPS ANTIVIRUS DE L'HERPÈS SIMPLEX ET LEURS PROCÉDÉS D'UTILISATION

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **05.01.2009 US 348550**

(43) Date of publication of application:
**26.10.2011 Bulletin 2011/43**

(73) Proprietor: **Development Center for Biotechnology**
**New Taipei City 22180 (TW)**

(72) Inventors:
• **LAI, Jiann-Shiun**
  **Xizhi City**
  **Taipei County 221 (TW)**
• **CHAN, Woan-Eng**
  **Xizhi City**
  **Taipei County 221 (TW)**

(74) Representative: **Green, Katherine et al**
**Mewburn Ellis LLP**
**City Tower**
**40 Basinghall Street**
**London EC2V 5DE (GB)**

(56) References cited:
**WO-A2-2005/011580**

• **CONNOLLY SARAH A ET AL: "Potential nectin-1 binding site on herpes simplex virus glycoprotein D", JOURNAL OF VIROLOGY, THE AMERICAN SOCIETY FOR MICROBIOLOGY, US, vol. 79, no. 2, 1 January 2005 (2005-01-01), pages 1282-1295, XP002507621, ISSN: 0022-538X, DOI: 10.1128/JVI.79.2.1282-1295.2005**
• **CHEN JIANMIN ET AL: "Prevention of genital herpes in a guinea pig model using a glycoprotein D-specific single chain antibody as a microbicide", VIROLOGY JOURNAL, BIOMED CENTRAL, LONDON, GB, vol. 1, no. 1, 23 November 2004 (2004-11-23), page 11, XP021010840, ISSN: 1743-422X, DOI: 10.1186/1743-422X-1-11**
• **MUGGERIDGE M I ET AL: "Antigenic and functional analysis of a neutralization site of HSV-1 glycoprotein D", VIROLOGY, ELSEVIER, AMSTERDAM, NL, vol. 174, no. 2, 1 February 1990 (1990-02-01), pages 375-387, XP026403872, ISSN: 0042-6822 [retrieved on 1990-02-01]**
• **MARTINA I MUGGERIDGE ET AL: "Antigenic Analysis of a Major Neutralization Site of Herpes Simplex Virus Glycoprotein D, Using Deletion Mutants and Monoclonal Antibody-Resistant Mutants", JOURNAL OF VIROLOGY, vol. 62, no. 9, 1 September 1988 (1988-09-01), pages 3274-3280, XP055051653,**

**(Cont. next page)**

- A. C. MINSON ET AL: "An Analysis of the Biological Properties of Monoclonal Antibodies against Glycoprotein D of Herpes Simplex Virus and Identification of Amino Acid Substitutions that Confer Resistance to Neutralization", JOURNAL OF GENERAL VIROLOGY, vol. 67, no. 6, 1 June 1986 (1986-06-01), pages 1001-1013, XP055051654, ISSN: 0022-1317, DOI: 10.1099/0022-1317-67-6-1001
- CONNOLLY S.A. ET AL.: 'Potential Nectin-I Binding Site on Herpes Simplex Virus Glycoprotein D.' JOURNAL OF VIROLOGY. vol. 79, no. 2, 2005, pages 1282 - 1295, XP002507621
- NICOLA A.V. ET AL.: 'Monoclonal Antibodies to Distinct Sites on Herpes Simplex Virus (HSV) Glycoprotein D Block HSV Binding to HVEM.' JOURNAL OF VIROLOGY. vol. 72, no. 5, 1998, pages 3595 - 3601, XP002132899
- EISENBERG R.J. ET AL.: 'Localization of Epitopes of Herpes Simplex Virus Type 1 Glycoprotein D.' JOURNAL OF VIROLOGY. vol. 53, no. 2, 1985, pages 634 - 644, XP008140787
- CHENG-CHUNG LEE ET AL: "Structural basis for the antibody neutralization of Herpes simplex virus", ACTA CRYSTALLOGRAPHICA SECTION D: BIOLOGICAL CRYSTALLOGRAPHY., vol. 57, no. 10, 20 September 2013 (2013-09-20), pages 122-1945, XP055222867, DK ISSN: 0907-4449, DOI: 10.1107/S0907444913016776
- Gary H Cohen ET AL: "Localization of discontinuous epitopes of herpes simplex virus glycoprotein D: use of a nondenaturing ("native" gel) system of polyacrylamide gel electrophoresis coupled with Western blotting", Journal of Virology, vol. 60, 1 October 1986 (1986-10-01), pages 157-166, XP055222870, UNITED STATES

<u>Remarks:</u>
The file contains technical information submitted after the application was filed and not included in this specification

## Description

Background of the Invention

Herpes simplex virus (HSV) is a member of the herpes virus family, which causes infection in humans. HSV infection results in a number of distinct medical disorders, depending on the infection sites. Infections in the mouth, face, hand, and genitals generally do not cause severe complications. However, infections in the eye, central nervous system, and brain can be life-threatening. Patients with immature or suppressed immune systems, such as newborns, transplant recipients, and HIV carriers, are prone to severe complications from HSV infections.

Several approaches are currently available for treating HSV infection, including antiviral medication and vaccine. However, there is still a need for the development of a drug that prevents or treats HSV infections.

Connolly et al. (J Virol. 2005 79(2): 1282-95) describes a region of HSV glycoprotein D (gD) that is a potential nectin-1 binding site. The authors also disclose that the binding site for the mouse monoclonal antibody, DL11, partially overlaps with this potential netrin-1 binding site. WO 2005/011580 A2 discloses a DL-11-based single chain antibody and describes its use in protecting animals against HSV type 1 and type 2 genital herpes.

Summary of the Invention

The present invention is based on the identification of human anti-HSV antibodies that exhibit high efficacy in inhibiting reproduction of HSV. The antibodies of the invention bind to a conformational epitope on the glycoprotein D (gD) of HSV-1 and HSV-2.

The invention provides antibodies, vectors, host cells, pharmaceutical compositions, methods of producing antibodies and antibodies for use in a method of treatment, as set out in the claims.

The invention provides an antibody that specifically binds to the gD of HSV (e.g., an antibody that specifically binds to the gD of HSV-1 and HSV-2). This antibody contains (i) a heavy chain variable region (VH) and a light chain variable region comprising the three complementarity determining regions (CDRs) of the VH of scFv E317, as shown in SEQ ID NO:1, and a light chain variable region (VL) comprising the three CDRs of the VL of scFv E317, as shown in SEQ ID NO:2; (ii) a VH comprising the three CDRs of the VH of scFv E425, as shown in SEQ ID NO:3, and a VL comprising the three CDRs of the VL of scFv E425, as shown in SEQ ID NO:4; or (iii) a VH comprising the three CDRs from SEQ ID NO:41 and a light chain variable region comprising three CDRs from SEQ ID NO: 42.

Disclosed herein is an isolated antibody (e.g., human antibody) or polypeptide that specifically binds to the gD of herpes simplex virus-1 (HSV-1) and herpes simplex virus-2 (HSV-2), wherein the antibody or the polypeptide binds to a conformational epitope on the glycoprotein D, and the epitope comprises amino acids Y38, D215, P221, and R222 shown in SEQ ID NO:44 or SEQ ID NO:45 or the amino acids on the gD corresponding to amino acids Y38, D215, P221, and R222 shown in SEQ ID NO:44 or SEQ ID NO:45. Also disclosed here is an antibody that binds to or interacts with amino acids 35-40 and 215-222 shown in SEQ ID NO:44 or SEQ ID NO:45 or the amino acids corresponding to the amino acids 35-40 and 215-222 shown in SEQ ID NO:44 or SEQ ID NO:45 on the gD.

Another aspect of the invention relates to a pharmaceutical composition comprising an antibody of the invention and a pharmaceutically acceptable carrier.

Another aspect of the present invention relates to an isolated nucleic acid comprising a nucleotide sequence encoding an antibody of the invention. Disclosed herein is a nucleic acid encoding any of the above-described $V_H$ and $V_L$ regions. In one example, this nucleic acid includes a nucleotide sequence encoding SEQ ID NO:1, amino acids 3-124 of SEQ ID NO:1, SEQ ID NO:2, amino acids 1-108 of SEQ ID NO:2, SEQ ID NO:3, amino acids 3-124 of SEQ ID NO:3, SEQ ID NO:4, amino acids 1-108 of SEQ ID NO:4, SEQ ID NO:41, or SEQ ID NO:42. Preferably, the nucleic acid includes a nucleotide sequence that encodes both SEQ ID NOs:1 and 2, encodes both amino acids 3-124 of SEQ ID NO:1 and amino acids 1-108 of SEQ ID NO:2, encodes both SEQ ID NOs:3 and 4, encodes both amino acids 3-124 of SEQ ID NO:3 and amino acids 1-108 of SEQ ID NO:4, or encodes both SEQ ID NOs:41 and 42. The invention also provides a vector (such as an expression vector) or a host cell comprising one or more nucleic acids of the invention.

The invention also provides methods for producing an antibody of the invention comprising culturing a host cell described herein that produces the antibody and recovering the antibody or the fragment form the cell culture. Mammalian cells (such as COS, HeLa, and CHO cells) and non-mammalian cells including prokaryotes (such as E. coli) and yeast may be used as host cells for producing the antibodies.

In yet another aspect, this invention features an antibody of the invention for use in a method for treating or preventing HSV infection by administering to a subject in need thereof an effective amount of the antibody of this invention. The antibody can also be used for the manufacture of a medicament for treating HSV infection. The term "treating" as used herein refers to the application or administration of a composition including one or more active agents to a subject, who is infected with HSV or at risk for HSV infection, with the purpose to cure, heal, alleviate, relieve, alter, remedy, ameliorate, improve, or affect the infection, the symptoms of the infection, or the predisposition toward the infection. "An effective amount" as used herein refers to the amount of each active agent required to confer therapeutic effect on the subject, either alone or in combination with one or more other active agents. Effective amounts vary, as recognized by those skilled in the art, depending on route of administration, excipient choice, and co-usage with other active agents. The antibody may be administered prior to or after exposure to a HSV (such as HSV-1 or HSV-2).

Also within the scope of this invention is a method of diagnosing HSV infection in a subject with an anti-HSV antibody of the invention. This method includes (a) contacting the antibody with a biological sample obtained from the subject and suspected of containing HSV viral particles or proteins, and (b) determining an antigen-antibody reaction. Detection of such a reaction indicates presence of HSV in the sample.

The details of one or more embodiments of the invention are set forth in the description below. Other features or advantages of the present invention will be apparent from the following detailed description of several embodiments, and also from the appended claims.

Brief Description of the Drawings

FIG. 1 shows the results on the protection from HSV lethality in HSV1-infected SCID mice by multiple mAb E317 dosage administrations at dosages of 1.5 mpk, 5 mpk, or 15 mpk. mAb E317 was administered five times at a five-day interval starting at 24 hours prior to virus inoculation.

FIG. 2 shows the results on the protection from HSV lethality in HSV1-infected SCID mice by a single mAb E317 injection at the dosage of 1.5 mpk, 5 mpk, or 15 mpk. mAb E317 was administered at 24 hours prior to virus inoculation.

FIG. 3 shows the results on the protection from HSV lethality in HSV1-infected SCID mice by a single mAb E317 injection at 15 mpk at 24 hours prior to virus inoculation, on the same day as virus inoculation, or 24 hours after virus inoculation.

FIG. 4 shows the results of the identification of the regions in glycoprotein D ("gD") important for epitope recognition by mAb E317 through immunoprecipitation assays. "+" indicates that binding of the antibody to the mutant gD was detected; "-" indicates that binding of the antibody to the mutant gD was not detected.

FIG. 5 shows the results of the identification of the amino acids in glycoprotein D ("gD") important for epitope recognition by mAb E317 through immunoprecipitation assays. "+" indicates that binding of the antibody to the mutant gD was detected; "-" indicates that binding of the antibody to the mutant gD was not detected.

Detailed Description of the Invention

Described herein are antibodies and polypeptides that bind to the glycoprotein D of HSV-1 and HSV-2 and neutralize HSV-1 and HSV-2. These antibodies and polypeptides bind to a non-linear conformational epitope on the gD, wherein the epitope comprises amino acids D215, P221, and R222 shown in SEQ ID NO:44 or SEQ ID NO:45, or the amino acids on the gD corresponding to amino acids D215, P221, and R222 shown in SEQ ID NO:44 or SEQ ID NO:45. In some instances, the epitope further comprises amino acid Y38 or the amino acid on the gD corresponding to Y38 shown in SEQ ID NO:44 or SEQ ID NO:45. In some instances, the antibody binds to or interacts with amino acids 35-40 and 215-222 shown in SEQ ID NO:44 or SEQ ID NO:45 or the amino acids on the gD corresponding to the amino acids 35-40 and 215-222 shown in SEQ ID NO:44 or SEQ ID NO:45.

It was generally believed that HSV entry into cells requires the interaction of gD with one of several potential entry receptors: (a) herpes virus entry mediator (HVEM), a member of the TNF receptor family; (b) nectin-1, a member of the immunoglobulin superfamily; and (c) 3-O-sulfated heparan sulphate. See Reske et al., Rev. Med. Virol. 17:205-215, 2007. HSV-1 gD is a type I membrane glycoprotein consisting of 369 amino acids within an N-terminal ectodomain of 316 amino acids. The protein's ectodomain core is of a V-like domain of the immunoglobulin fold (residues 56-184). Within the N-terminal extension to the core, a 37 residue hairpin structure (bending at residue 21) forms the entire site which contacts the receptor HVEM. In the absence of HVEM, the N-terminus adopts an extended and flexible conformation. Without wishing to be bound by theory, the antibodies described herein may bind to amino acids of the gD that interact with the N-terminal sequences when the hairpin structure of the gD is formed, and the binding of the antibody to the gD may prevent the formation of the hairpin structure. It has been demonstrated that antibodies that specifically bind this portion of the gD exhibits high efficacy in inhibiting reproduction of HSV in vitro and in vivo.

The antibodies or polypeptides described herein may have one or more of the following characteristics: (a) specifically bind to the gD of HSV-1 and HSV-2; (b) bind to a non-linear conformation epitope of the gD; (c) bind to an epitope including at least amino acids D215, P221, and R222 shown in SEQ ID NO:44 or SEQ ID NO:45 or the amino acids on the gD corresponding to amino acid residues D215, P221, and R222 shown in SEQ ID NO:44 or SEQ ID NO:45; (d) bind to an epitope including amino acids Y38, D215, P221, and R222 shown in SEQ ID NO:44 or SEQ ID NO:45 or the amino acids on the gD corresponding to amino acid residues Y38, D215, P221, and R222 shown in SEQ ID NO:44 or SEQ ID NO:45; (e) bind to or interact with amino acids 35-40 and 215-222 shown in SEQ ID NO:44 or SEQ ID NO:45 or the amino acids on the gD corresponding to the amino acids 35-40 and 215-222 shown in SEQ ID NO:44 or SEQ ID NO:45; (f) binding of the antibodies or polypeptides to the gD is disulfide bond dependent; (g) do not bind the gD if amino acids 224-240 shown in SEQ ID NO:44 or 45 or amino acids on the gD corresponding to the amino acids shown in SEQ ID NO:44 or 45 are deleted; (h) do not bind to the gD if amino acids 27-43 shown in SEQ ID NO:44 or 45 or amino acids on the gD corresponding to amino acids 27-43 shown in SEQ ID NO:44 or 45 are deleted; (i) having an $IC_{50}$ between about 1 nM to about 10 nM in inhibiting HSV-1 and/or HSV-2 reproduction in a plaque reduction assay; and (j) prevent or treat HSV infection in a subject.

Glycoprotein D of different strains of HSV-1 and HSV-2 may have variations in amino acid sequences. The alignment for the amino acid sequences of the gD from KOS (a strain of HSV-1) (SEQ ID NO:44) and the gD from HG52 (a strain of HSV-2) (SEQ ID NO:45) is shown below. One skilled in the art can easily identify amino acid residues in a glycoprotein D that correspond to the positions shown in SEQ ID NO:44 or 45.

```
                            10        20        30        40        50
gD-KOS    KYALADASLKMADPNRFRGKDLPVLDQLTDPPGVRRVYHIQAGLPDPFQP
gD-HG52   KYALADPSLKMADPNRFRGKNLPVLDQLTDPPGVKRVYHIQPSLEDPFQP


                            60        70        80        90        100
gD-KOS    PSLPITVYYAVLERACRSVLLNAPSEAPQIVRGASEDVRKQPYNLTIAWF
gD-HG52   PSIPITVYYAVLERACRSVLLHAPSEAPQIVRGASDEARKHTYNLTIAWY


                            110       120       130       140       150
gD-KOS    RMGGNCAIPITVMEYTECSYNKSLGACPIRTQPRWNYYDSFSAVSEDNLG
gD-HG52   RMGDNCAIPITVMEYTECPYNKSLGVCPIRTQPRWSYYDSFSAVSEDNLG


                            160       170       180       190       200
gD-KOS    FLMHAPAFETAGTYLRLVKINDWTEITQFILEHRAKGSCKYALPLRIPPS
gD-HG52   FLMHAPAFETAGTYLRLVKINDWTEITQFILEHRARASCKYALPLRIPPA

                            210       220       230       240       250
gD-KOS    ACLSPQAYQQGVTVDSIGMLPRFIPENQRTVAVYSLKIAGWHGPKAPYTS
gD-HG52   ACLTSKAYQQGVTVDSIGMLPRFIPENQRTVALYSLKIAGWHGPKPPYTS

                            260       270       280       290       300
gD-KOS    TLLPPELSETPNATQPELAPEDPEDSALLEDPVGTVAPQIPPNWHIPSIQ
gD-HG52   TLLPPELSDTTNATQPELVPEDPEDSALLEDPAGTVSSQIPPNWHIPSIQ

                            310       320       330       340       350
gD-KOS    DAATPYHPPATPNNMGLIAGAVGGSLLAALVICGIVYWMHRRTRKAPKRI
gD-HG52   DVA-PHHAPAAPSNPGLIIGALAGSTLAVLVIGGIAFWVRRRAQMAPKRL

                            360       370       380       390       400
gD-KOS    RLPHIREDDQPSSHQPLFY  (SEQ ID NO:44)
gD-HG52   RLPHIRDDDAPPSHQPLFY  (SEQ ID NO:45)
```

The term "antibody" is meant to include intact naturally-occurring antibodies and their fragments (e.g., Fab and F(ab')$_2$) and genetically modified antibodies (e.g., scFv antibodies, chimeric antibodies, diabodies, and dual variable domain immunoglobulins). In some embodiments, the antibody is a human antibody. In some instances, the antibody is a humanized antibody.

An "isolated" molecule or material (e.g., an antibody and a nucleic acid) is one which has been identified and separated and/or recovered from a component of its natural environment. In some instances the molecule or material is purified. For example, the material may be at least 90% pure (i.e., free from contaminants), at least 95% pure, or at least 99% pure.

As used herein and in the appended claims, the singular forms "a", "an", and "the" include plural reference unless the context clearly indicates otherwise. It is understood that aspect and variations of the invention described herein include "consisting" and/or "consisting essentially of" aspects and variations.

Also disclosed herein are human antibodies, e.g., E317, E425 and Y571, that specifically bind to both herpes simplex virus 1 (HSV-1) and herpes simplex virus 2 (HSV-2), in particular, their glycoprotein D.

The E317 antibody contains a $V_H$ fragment, which or a portion of which has the amino acid sequence shown in residues 3-124 of SEQ ID NO:1; and a $V_L$ fragment, which or a portion of which has the amino acid sequence shown in residues 1-108 of SEQ ID NO:2. These two amino acid sequences are shown below:

**Amino acid sequence of the E317 $V_H$ fragment (SEQ ID NO:1)**

```
MAQVTLKQSGAEVKKPGSSVKVSCTASGGTLRTYGVSWVRQAPGQGLEWLGRTIPLFGKTDY
AQKFQGRVTITADKSMDTSFMELTSLTSEDTAVYYCARDLTTLTSYNWWDLWGQGTLVTVSS
```

[* The underlined regions in the above sequence refer to complementarity-determining regions (CDRs)].

**Amino acid sequence of the E317 $V_L$ fragment (SEQ ID NO:2)**

```
EIVLTQSPGTLSLSPGERATLSCRASQSVTSSQLAWYQQKPGQAPRLLISGASNRATGIPDR
FSGSGSGTDFTLTISRLEPEDFAVYYCQQYGSSPTFGGGTKVEIKRAA
```

[* The underlined regions in the above sequence refer to CDRs.]

The E425 antibody contains a $V_H$ fragment, which or a portion of which has the amino acid sequence shown in residues 3-124 of SEQ ID NO:3 and a $V_L$ fragment, which or a portion of which has the amino acid sequence shown in residues 1-108 of SEQ ID NO:4. Both of the sequences are shown below:

**Amino acid sequence of the E425 $V_H$ fragment (SEQ ID NO:3)**

```
MAQVQLQQSGAGVKKPGSSVRVSCSASGGTLRTYALSWVRQVPGQGFEWMGRIIPMFGKTDY
AQKFQGRLSITADKSMDTGFMELTSLTSEDTAVYYCARDLTTLTSYNWLDIWGQGTLVTVSS
```

[* The underlined regions in the above sequence refer to CDRs.]

**Amino acid sequence of the E425 $V_L$ fragment (SEQ ID NO:4)**

```
ETTLTQSPGTLSLSPGERATLSCRASQSVSSNYLAWYQKKPGQAPRLLIYGASSRATGIPDR
FSGSGSGTDFTLTINRLEPEDFAVYYCQQYGRSPTFGQGTKVEIKRAA
```

[* The underlined regions in the above sequence refer to CDRs.]

The Y571 antibody contains a $V_H$ fragment, which or a portion of which has the amino acid sequence of SEQ ID NO:41 and a $V_L$ fragment, which or a portion of which has the amino acid sequence of SEQ ID NO:42. Both of the sequences are shown below:

**Amino acid sequence of the Y571 $V_H$ fragment (SEQ ID NO:41)**

```
QVQLQQSGAEVKKPGSSVKVSCKASGGTLRTYGVSWVRQAPGQGLEWLGGTIPLFGKTDYAQ
KFQGRVTITADKSMDTSFMELTSLTSEDTAVYYCARDLTTLTSYNWWDLWGQGTLVTVSS
```

[* The underlined regions in the above sequence refer to CDRs.]

**Amino acid sequence of the Y571 $V_L$ fragment (SEQ ID NO:42)**

```
ETTLTQSPGILSLSPGDRATLSCRASQSVGSVNLAWYQQRPGQAPRLLIHGASNRATGIPDR
FSGVGSGTDFTLTINRLEPDDFAVYYCQQYGTSPITFGQGTRLEIKR
```

[* The underlined regions in the above sequence refer to CDRs.]

Also disclosed herein are antibodies and polypeptides that compete with antibody scFv E317, scFv E425, and/or scFv Y571 for binding to the gD of HSV-1 and/or HSV-2. An antibody or polypeptide is considered competing with another antibody or polypeptide if the binding of one antibody or polypeptide to the gD is significantly inhibited by the presence of the second antibody or polypeptide at increasing concentrations. For example, the inhibition can be at least 50%, at least 60%, at least 70%, at least 80%, or at least 90%. As disclosed herein, some of the antibodies and polypeptides are able to neutralize HSV-1 and HSV-2 with substantially equivalent potency. The potency may be measured in vitro or in vivo. For example, the antibody or polypeptide may have an $IC_{50}$ from about 1 nM to about 10 nM (such as from about 1 nM to about 6 nM, or about 1 nM to about 5 nM) in the plaque assay described in Example 3.

Also disclosed herein are functional equivalents of the E317, E425, and Y571 antibody. Such a functional equivalent is an antibody that specifically binds to HSV glycoprotein D and has a $V_H$ fragment, which or a portion of which is at least 70% (e.g., 75%) identical to SEQ ID NO:1, SEQ ID NO:3, or SEQ ID NO:41, and a $V_L$ fragment, which or a portion of which is at least 70% (e.g., 75%) identical to SEQ ID NO:2, SEQ ID NO:4, or SEQ ID NO:42.

As used herein, "percent homology" of two amino acid sequences is determined using the algorithm described in Karlin and Altschul, Proc, Natl. Acad. Sci. USA 87:2264-2268, 1990, modified as described in Karlin and Altschul, Proc, Natl. Acad. Sci. USA 5873-5877, 1993. Such an algorithm is incorporated into the NBLAST and XBLAST programs of Altschul et al., J. Mol. Biol. 215:403-410, 1990. BLAST protein searches are performed with the XBLAST program, score = 50, wordlength = 3, to obtain amino acid sequences homologous to a reference polypeptide. To obtain gapped alignments for comparison purposes, Gapped BLAST is utilized as described in Altschul et al., Nucleic Acids Res. 25:3389-3402, 1997. When utilizing the BLAST and Gapped BLAST programs, the default parameters of the respective programs (e.g., XBLAST and NBLAST) are used. See www.ncbi.nlm.nih.gov.

The antibody of this invention can contain only the $V_H$ and $V_L$ fragments of E317, E425, or Y571 described above. It can be a single-chain antibody (scFv), in which the $V_H$; and $V_L$ fragments are connected either directly or via a linker (e.g., a peptide linker). In one example, the antibody is scFv E317, the amino acid sequence of which is shown below:

### Amino acid sequence of ScFv E317 (SEQ ID NO:5)

```
QVTLKQSGAEVKKPGSSVKVSCTASGGTLRTYGVSWVRQAPGQGLEWLGRTIPLFGKTDYAQ
KFQGRVTITADKSMDTSFMELTSLTSEDTAVYYCARDLTTLTSYNWWDLWGQGTLVTVSSGG
```

```
GGSGGGGSGGGGSEIVLTQSPGTLSLSPGERATLSCRASQSVTSSQLAWYQQKPGQAPRLLI
SGASNRATGIPDRFSGSGSGTDFTLTISRLEPEDFAVYYCQQYGSSPTFGGGTKVEIKR
```

(* The underlined region refers to the peptide linker connecting the $V_H$ and $V_L$ fragments.)

In another example, the antibody is scFv E425, having the amino acid of SEQ ID NO:6 shown below:

### Amino acid sequence of ScFv E425 (SEQ ID NO:6)

```
QVQLQQSGAGVKKPGSSVRVSCSASGGTLRTYALSWVRQVPGQGFEWMGRIIPMFGKTDYAQ
KFQGRLSITADKSMDTGFMELTSLTSEDTAVYYCARDLTTLTSYNWLDIWGQGTLVTVSSGG
GGSGGGGSGGGGSETTLTQSPGTLSLSPGERATLSCRASQSVSSNYLAWYQKKPGQAPRLLI
YGASSRATGIPDRFSGSGSGTDFTLTINRLEPEDFAVYYCQQYGRSPTFGQGTKVEIKR
```

(* The underlined region refers to the peptide linker connecting the $V_H$ and $V_l$, fragments.)

In another example, the antibody is scFv Y571, having the amino acid of SEQ ID NO:43 shown below:

**Amino acid sequence of ScFv Y571 (SEQ ID NO:43)**

```
QVQLQQSGAEVKKPGSSVKVSCKASGGTLRTYGVSWVRQAPGQGLEWLGGTIPLFGKTDYAQ
KFQGRVTITADKSMDTSFMELTSLTSEDTAVYYCARDLTTLTSYNWWDLWGQGTLVTVSSGG
GGSGGGGSGGGGSETTLTQSPGILSLSPGDRATLSCRASQSVGSVNLAWYQQRPGQAPRLLI
HGASNRATGIPDRFSGVGSGTDFTLTINRLEPDDFAVYYCQQYGTSPITFGQGTRLEIKR
```

(* The underlined region refers to the peptide linker connecting the $V_H$ and $V_l$, fragments.)

The invention provides antibodies comprising a heavy chain variable region comprising three CDRs from SEQ ID NO:1 and a light chain variable region comprising three CDRs from SEQ ID NO:2. The invention also provides antibodies comprising the heavy chain variable region comprising the amino acid residues 3-124 shown in SEQ ID NO:1 andthe light chain variable region comprising the amino acid residues 1-108 shown in SEQ ID NO:2. The invention also provides antibodies comprising a heavy chain variable region comprising three CDRs from SEQ ID NO:3 and a light chain variable region comprising three CDRs from SEQ ID NO:4. The invention also provides antibodies comprising the heavy chain variable region comprising the amino acid residues 3-124 shown in SEQ ID NO:3 and the light chain variable region comprising the amino acid residues 1-108 shown in SEQ ID NO:4. The invention also provides antibodies comprising a heavy chain variable region comprising three CDRs from SEQ ID NO:41 and a light chain variable region comprising three CDRs from SEQ ID NO:42. The invention also provides antibodies comprising the heavy chain variable region comprising the amino acid sequence shown in SEQ ID NO:41 and the light chain variable region comprising the amino acid sequence shown in SEQ ID NO:42. The invention also provides antibodies comprising the amino acid sequence shown in SEQ ID NO:5, 6, or 43.

As used herein, a "complementarity determining region" or "CDR" includes a CDR by any definition, such as a Kabat CDR (Kabat et al., Sequences of Proteins of Immunological Interest, 5th Ed. Public Health Service, National Institutes of Health, Bethesda, MD. (1991)), a Chothia CDR (Chothia and Lesk, J. Mol. Biol. 196:901-917 (1987)), and combined CDRs. In some instances, the CDR is an abbreviated CDR which includes stretches of CDR residues that include all the specificity determining residues (SDRs) (Kashmiri et al., Methods 36:25-34, 2005). In some instances, the invention provides antibodies comprising all the SDRs from antibody ScFv E317, ScFv E425, or ScFv Y571.

The antibody of this invention can also be a whole immunoglobulin molecule, in which the V H and VL fragments are respectively linked to a heavy chain constant region and a light chain constant region of an immunoglobulin, e.g., human IgGl, IgG2a, IgG2b, IgG3, IgG4, IgM, IgE, IgD, IgAl, and Ig A2.

The antibodies described herein may be generated by screening an antibody library (such as a phage display library) and identify antibodies that have the desired binding properties using methods known in the art and described herein. See, e.g., Examples 1-5.

Any of the above-described antibodies can be made using conventional recombinant technology. For example, the antibody may be produced in a host cell by expressing a nucleic acid encoding the antibody, and recovering the antibody from the cell culture. For example, the E317, E425, or Y571 antibody can be prepared by expressing polypeptides containing residues 3-124 of SEQ ID NO:1, residues 3-124 of SEQ ID NO:3, or SEQ ID NO:41 for the V H, and/or residues 1-108 of SEQ ID NO:2, residues 1-108 of SEQ ID NO:4, or SEQ ID NO:42 for the VL in host cells from one or two expression cassettes containing the nucleotide sequences coding for the polypeptides. The VH and VL fragments can be made as two separate polypeptides and then refolded together to form an antibody. Alternatively, the two fragments are produced as parts of a single polypeptide.

Functional equivalents of E317, E425, or Y571 can be produced by introducing mutations in their V H and VL fragments, preferably, in the frame regions (FRs). It is well known that the CDRs of an antibody determine its antigen specificity. Thus, mutations, particularly conservative mutations, in the FRs of E317, E425, or Y571 normally would not affect its binding activity to HSV. The antigen specificity of a functional equivalent thus prepared can be confirmed using methods known in the art, e.g., ELISA, immunoprecipitation, or Western-blot analysis.

Both of the antibodies described herein and their encoding nucleic acids are useful in treating HSV infection or reducing HSV reproduction in a human subject who needs the treatment, such as a patient infected with HSV or a patient having a weakened immune system (e.g., infants at risk for congenital HSV infection, pregnant women, organ transplant recipients, cancer patients, leukemia patients, and HIV carriers).

To practice the treatment mentioned above, any of the antibody of this invention or the encoding nucleic acid(s) can be suspended in a pharmaceutically-acceptable carrier (e.g., physiological saline) to form a pharmaceutical composition and administered via a conventional route. The carrier contained in the pharmaceutical composition must be "acceptable" in the sense that it is compatible with the active ingredient of the composition, and preferably, capable of stabilizing the active ingredient and not deleterious to the subject to be treated. The pharmaceutical composition can be administered orally or by intravenous infusion, or injected or implanted subcutaneously, intramuscularly, intrathecally, intraperitoneally, intrarectally, intravaginally, intranasally, intragastrically, intratracheally, or intrapulmonarily. When the encoding nucleic acid(s) is used, it can be delivered via a live vector, such as Salmonella, BCG, adenovirus, poxvirus or vaccinia.

The pharmaceutical composition mentioned above can be formulated into dosage forms based on the intended administration routes utilizing conventional methods. For example, it can be formulated in a capsule, a gel seal, or a tablet for oral administration. Capsules can contain any standard pharmaceutically acceptable materials such as gelatin or cellulose. Tablets can be formulated in accordance with conventional procedures by compressing mixtures of the composition with a solid carrier and a lubricant. Examples of solid carriers include starch and sugar bentonite. The capsules or tablets can further contain a binder (e.g., lactose or mannitol), conventional filler, and tableting agent to form hard shell capsules or tablets.

The pharmaceutical composition can also be formulated into parenteral dosage forms, include aqueous solutions, isotonic saline or 5% glucose of the active agent, or other well-known pharmaceutically acceptable excipient. Cyclodextrins, or other solubilizing agents well known to those familiar with the art, can be utilized as pharmaceutical excipients for delivery of the therapeutic agent.

Injectable compositions containing either the antibody or its encoding nucleic acid(s) may contain various carriers such as vegetable oils, dimethylactamide, dimethyformamide, ethyl lactate, ethyl carbonate, isopropyl myristate, ethanol, and polyols (glycerol, propylene glycol, liquid polyethylene glycol, and the like). For intravenous injection, water soluble antibodies can be administered by the drip method, whereby a pharmaceutical formulation containing the antibody and a physiologically acceptable excipients is infused. Physiologically acceptable excipients may include, for example, 5% dextrose, 0.9% saline, Ringer's solution or other suitable excipients. Intramuscular preparations, e.g., a sterile formulation of a suitable soluble salt form of the antibody, can be dissolved and administered in a pharmaceutical excipient such as Water-for-Injection, 0.9% saline, or 5% glucose solution.

To facilitate delivery, the antibody described herein or its encoding nucleic acid(s) can be conjugated with a chaperon agent. As used herein, "conjugated" means two entities are associated, preferably with sufficient affinity that the therapeutic benefit of the association between the two entities is realized. Conjugated includes covalent or noncovalent bonding as well as other forms of association, such as entrapment of one entity on or within the other, or of either or both entities on or within a third entity (e.g., a micelle).

The chaperon agent can be a naturally occurring substance, such as a protein (e.g., human serum albumin, low-density lipoprotein, or globulin), carbohydrate (e.g., a dextran, pullulan, chitin, chitosan, inulin, cyclodextrin or hyaluronic acid), or lipid. It can also be a recombinant or synthetic molecule, such as a synthetic polymer, e.g., a synthetic polyamino acid. Examples of polyamino acids include polylysine (PLL), poly L-aspartic acid, poly L-glutamic acid, styrene-maleic acid anhydride copolymer, poly(L-lactide-co-glycolied) copolymer, divinyl ether-maleic anhydride copolymer, N-(2-hydroxypropyl) methacrylamide copolymer (HMPA), polyethylene glycol (PEG), polyvinyl alcohol (PVA), polyurethane, poly(2-ethylacryllic acid), N-isopropylacrylamide polymers, and polyphosphazine.

In one example, the chaperon agent is a micelle, liposome, nanoparticle, or microsphere, in which the oligonucleotide is encapsulated. Methods for preparing such a micelle, liposome, nanoparticle, or microsphere are well known in the art. See, e.g., US Patents 5,108,921; 5,354,844; 5,416,016; and 5,527,5285.

In another example, the chaperon agent serves as a substrate for attachment of one or more of a fusogenic or condensing agent.

A fusogenic agent is responsive to the local pH. For instance, upon encountering the pH within an endosome, it can cause a physical change in its immediate environment, e.g., a change in osmotic properties which disrupts or increases the permeability of the endosome membrane, thereby facilitating release of the antibody or nucleic acid(s) into host cell's cytoplasm. A preferred fusogenic agent changes charge, e.g., becomes protonated at a pH lower than a physiological range (e.g., at pH 4.5-6.5). Fusogenic agents can be molecules containing an amino group capable of undergoing a change of charge (e.g., protonation) when exposed to a specific pH range. Such fusogenic agents include polymers having polyamino chains (e.g., polyethyleneimine) and membrane disruptive agents (e.g., mellittin). Other examples include polyhistidine, polyimidazole, polypyridine, polypropyleneimine, and a polyacetal substance (e.g., a cationic polyacetal).

A condensing agent interacts with the antibody/nucleic acid(s), causing it to condense (e.g., reduce the size of the oligonucleotide), thus protecting it against degradation. Preferably, the condensing agent includes a moiety (e.g., a charged moiety) that interacts with the oligonucleotide via, e.g., ionic interactions. Examples of condensing agents include polylysine, spermine, spermidine, polyamine or quarternary salt thereof, pseudopeptide-polyamine, peptidomimetic polyamine, dendrimer polyamine, arginine, amidine, protamine, cationic lipid, cationic porphyrin, and alpha helical peptide.

The dosage of the antibody or the encoding nucleic acid(s) required in the method of this invention depends on the choice of the route of administration; the nature of the formulation; the nature of the subject's illness; the subject's size, weight, surface area, age, and sex; other drugs being administered; and the judgment of the attending physician. Suitable dosages are in the range of 0.01-100.0 mg/kg. Wide variations in the needed dosage are to be expected in view of the variety of compositions available and the different efficiencies of various routes of administration. For example, oral administration would be expected to require higher dosages than administration by intravenous injection. Variations in these dosage levels can be adjusted using standard empirical routines for optimization as is well understood in the art. Encapsulation of the composition in a suitable delivery vehicle (e.g., polymeric microparticles or implantable devices) may increase the efficiency of delivery, particularly for oral delivery.

The efficacy of the antibody of this invention for treating HSV infection can be evaluated both in vitro and in vivo. Briefly, the antibody can be tested for its ability to inhibit viral protein production or virus reproduction in vitro. For in vivo studies, the antibody can be injected into an animal (e.g., a mouse model) and its therapeutic effects are then accessed. Based on the results, an appropriate dosage range and administration route can be determined.

Also within the scope of this invention is a method for diagnosing HSV infection in a human subject with the antibody of this invention. In this method, a test sample (e.g., a blood sample or an oral or genital tissue sample) is obtained from a human subject suspected of having HSV infection and then examined for presence of HSV viral proteins using the antibody of this invention via an immunoassays suitable for detecting an antibody-antigen reaction. For example, the test sample can be incubated with an anti-HSV antibody as described herein under conditions suitable for formation of an antibody-antigen complex and the complex, if formed, is detected by a conventional immunoassay, e.g., ELISA, immunoprecipitation, and immunohistochemistry.

Also disclosed herein are kits comprising an antibody or an antigen-binding fragment described herein. In some instances, the kits comprise one or more containers comprising the antibody or the fragment described herein and instructions for use in accordance with any of the methods of the invention described herein. In some instances, the instructions comprise a description of administration of the antibody or the fragment to prevent or treat HSV infection in a subject. In some instances, the instructions comprise a description of using the antibody or the fragment for detecting the presence of HSV in a sample.

Without further elaboration, it is believed that one skilled in the art can, based on the above description, utilize the present invention to its fullest extent. The following specific embodiments are, therefore, to be construed as merely illustrative, and not limitative of the remainder of the disclosure in any way whatsoever.

EXAMPLE 1 : Isolation of Human Anti-HSV scFv Antibodies from A Mixed Phage scFv Library

A scFv phage display library was generated using RNAs isolated from 50 healthy Asian adults, following the procedure described in Clackson et al., Nature, 352:624-628 (1991). Briefly, mRNAs were purified from B lymphocytes isolated from the 50 healthy Asian adults. cDNAs corresponding to the $V_H$ domains of immunoglobulin proteins were amplified from these mRNAs via RT-PCR, using the following primers:

$V_H$back: HuVH1abacksfi: 5' - GTCCTCGCAACTGCGGCCCAGCCGGCCATGGCCCAGGTGCAGCTGGTGSARTCTGG-3' (SEQ ID NO:7)
HuVH2abacksfi:5'GTCCTCGCAACTGCGGCCCAGCCGGCCATGGCCCAGGTCAACTTAAGGGAGTCTGG-3' ID NO: 8)

HuVH3abacksfi:5'-GTCCTCGCAACTGCGGCCCAGCCGGCCATGGCCGAGGTGCAGC TGKTGGAGWCY-3' (SEQ ID NO:9)

HuVH4abacksfi:5'-GTCCTCGCAACTGCGGCCCAGCCGGCCATGGCCCAGGTGCAGC TGCAGGAGTCSG-3' (SEQ ID NO:10)

HuVH5abacksfi:5'-GTCCTCGCAACTGCGGCCCAGCCGGCCATGGCCGAGGTGCAGC TGTTGCAGTCTGC-3' (SEQ ID NO:11)

HuVH6abacksfi:5'-GTCCTCGCAACTGCGGCCCAGCCGGCCATGGCCCAGGTACAGC TGCAGCAGTCA-3' (SEQ ID NO:12)

HuVH14abacksfi:5'-GTCCTCGCAACTGCGGCCCAGCCGGCCATGGCCCAGRTCACC TTGAAGGAGTCTG -3' (SEQ ID NO:13)

HuVH16abacksfi:5'-GTCCTCGCAACTGCGGCCCAGCCGGCCATGGCCCAGGTGCAG CTACAGCAGTGGG-3' (SEQ ID NO:14)

$J_H$for:

HuJH1-2for: 5'-TGAGGAGACGGTGACCAGGGTGCC-3' (SEQ ID NO:15)
HuJH3 for: 5'-TGAAGAGACGGTGACCATTGTCCC-3' (SEQ ID NO:16)
HuJH4-5 for: 5'-TGAGGAGACGGTGACCAGGGTTCC-3' (SEQ ID NO:17)
HuJH6 for: 5'-TGAGGAGACGGTGACCGTGGTCCC-3' (SEQ ID NO:18)

cDNAs corresponding to the $V_L$ domains of immunoglobulins were amplified using the primers shown below.

$V_K$back:

HuVK1a back: 5'-GACATCCAGATGACCCAGTCTCC-3' (SEQ ID NO:19)
HuVK2a back: 5'-GATGTTGTGATGACTCAGTCTCC-3' (SEQ ID NO:20)
HuVK3a back" 5'-GAAATTGTGTTGACGCAGTCTCC-3' (SEQ ID NO:21)
HuVK4a back: 5'-GACATCGTGATGACCCAGTCTCC-3' (SEQ ID NO:22)
HuVK5a back: 5'-GAAACGACACTCACGCAGTCTCC-3' (SEQ ID NO:23)
HuVK6a back: 5'-GAAATTGTGCTGACTCAGTCTCC-3' (SEQ ID NO:24)

$I_K$for Not:

HuJK1 forNot: 5'-GAGTCATTCTCGACTTGCGGCCGCACGTTTGATTTCCACCTTGGTC CC-3' (SEQ ID NO:25)
HuJK2forNot: 5'-GAGTCATTCTCGACTTGCGGCCGCACGTTTGATCTCCAGCTTGGTCC C-3' (SEQ ID NO:26)
HuJK3forNot: 5'-GAGTCATTCTCGACTTGCGGCCGCACGTTTGATATCCACTTTGGTCC C-3' (SEQ ID NO:27)
HuJK4forNot: 5'-GAGTCATTCTCGACTTGCGGCCGCACGTTTGATCTCCACCTTGGTCC C-3' (SEQ ID NO:28)
HuJK5forNot: 5'-GAGTCATTCTCGACTTGCGGCCGCACGTTTAATCTCCAGTCGTGTCC C-3' (SEQ ID NO:29)

$V_\lambda$back:

HuVL1 back: 5'-CAGTCTGTGTTGACGCAGCCGCC-3' (SEQ ID NO:30)
HuVL2 back: 5'-CAGTCTGCCCTGACTCAGCCTGC-3' (SEQ ID NO:31)
HuVL3a back: 5'-TCCTATGTGCTGACTCAGCCACC-3' (SEQ ID NO:32)
HuVL3b back: 5'-TCTTCTGAGCTGACTCAGGACCC-3' (SEQ ID NO:33)
HuVL4 back: 5'-CACGTTATACTGACTCAACCGCC-3' (SEQ ID NO:34)
HuVL5 back: 5'-CAGGCTGTGCTCACTCAGCCGTC-3' (SEQ ID NO:35)
HuVL6 back: 5'-AATTTTATGCTGACTCAGCCCCA-3' (SEQ ID NO:36)

$J_\lambda$ for Not:

HuJL1forNot: 5'-GAGTCATTCTCGACTTGCGGCCGCACCTAGGACGGTGACCTTGGT CCC-3' (SEQ ID NO:37)
HuJL2-3forNot: 5'-GAGTCATTCTCGACTTGCGGCCGCACCTAGGACGGTCAGCTTGGT CCC-3' (SEQ ID NO:38)
HuJL4-5forNot: 5'-GAGTCATTCTCGACTTGCGGCCGCACCTAAAACGGTGAGCTGGGT CCC-3' (SEQ ID NO:39)

The $V_H$ cDNAs were randomly linked by PCR reactions with the $V_L$ cDNAs via a linker having the nucleotide sequence of 5'-GGTGGAGGCGGTTCAGGCGGAGGTGGCTCT GGCGGTGGCGGATCG -3' (SEQ ID NO:40) to form fragments encoding scFv antibodies. These fragments were cloned into pCANTAB 5E phagemid vector to produce the scFv phage display library.

Three additional scFv phage display libraries were constructed using mRNAs isolated from B lymphocytes of an Indian patient, B lymphocytes of a patient having severe acute respiratory syndrome, and spleen cells of a Taiwanese patient, following the procedures described above. These three libraries were combined with the library described above to form a mixed scFv phage display library, which was used to screen for anti-HCMV scFv antibodies. This mixed library, having a phage titer of $1 \times 10^{13}$ pfu/ml, was subjected to screening for clones that express scFv antibodies specific to HSV as follows.

First, phages displaying anti-HSV antibodies were enriched by five rounds of bio-panning as described below. An immunotube was coated with HSV-1 virons diluted in a coating buffer (50mM sodium bicarbonate, PH9.6 ,5 x $10^5$/ml) at 4° C overnight, washed three times with PBS containing 0.1% Tween , blocked with PBS containing 2% non-fat milk, and again washed three times with PBS containing 0.1% Tween. An aliquot of the mixed phage library was diluted in PBS containing 2% non-fat milk, and added to the immunotube coated with HSV-1. After a two-hour incubation, the immunotube was washed 10 times with PBS containing 0.1% Tween and then 10 times with PBS to remove unbound phages. The bound phages were eluted using 100mM triethylaine, neutralized with 1M Tris, PH7.4, and used to infect TG1 bacteria at 37° C for 30 minutes. The phage-infected TG1 cells were then placed on a plate containing 2x YT medium supplemented with amplicillin and glucose (2YT/amplicillin/glucose medium) and grew at 30°C overnight. A 2YT/amplicillin/glucose medium supplemented with glycerol was added on the plate and the TG1 cells were dispersed into the medium using a glass spreader. The TG1 cells thus collected were inoculated into a 2YT/amplicillin/glucose medium and cultured at 37° C, 250 rpm until the $OD_{600}$ value of the TG1 culture reached 0.5. After mixing with $5 \times 10^{10}$ pfu of M13KO7 helper phages, the TG1 culture was incubated at 37° C for 30 minutes and centrifuged at 2,000 x g for 10 minutes afterwards at room temperature. The cell pellet thus formed was re-suspended in 10 ml 2x YT medium supplemented with amplicillin and kanamycin and incubated at 30° C , 250 rpm overnight. The culture was centrifuged at 10,000 g for 20 minutes at 4° C to collect the resultant supernatant. PEG/NaCl was then added to the supernatant. An hour later, the supernatant was centrifuged to collect a pellet containing phage particles. The pellet was re-suspend in PBS and centrifuged to remove the remaining bacterial debris. The phage particles thus collected were again suspended in PBS to form the 1st round scFv phage library enriched in clones expressing anti-HSV antibodies.

An aliquot of this 1st round enriched library was subjected to additional four rounds of biopanning, following the procedures described above, to produce a phage library further enriched in phage clones expressing anti-HSV antibodies. This phage library was subjected to ELISA screening following the process described below to identify individual phage clones expressing anti-HSV single-chain antibodies.

An aliquot of the phage library was diluted and plated on 2 x YT medium supplemented with ampilicillin and glucose and incubated at 37° C overnight. 376 single colonies were picked and incubated separately in 2 x YT medium supplemented with ampicillin and glucose at 37° C, 250 rpm overnight. An aliquot of each of the cultures thus obtained was inoculated into a fresh 2 x YT medium supplemented with ampicillin and glucose and then mixed with $10^9$ pfu M13KO7helper phage. The mixtures were cultured at 37° C, 250 rpm for 1-2 hours, and then centrifuged at 14,000 rpm for 5 minutes at room temperature. The cell pellets, suspended in 2 x YT medium supplemented with ampicillin and kanamycin, were incubated at 30° C, 250 rpm overnight, and then centrifuged at 2000 g for 30 minutes at room temperature. The supernatants were subjected to the ELISA screening as follows.

A test multi-well microplate was coated with lysates of TG1 cells infected with HSV-1 and a control microplate was coated with lysates of E. coli cells transfected with vector pET-22b. The supernatants, each containing particles of one of the 376 phage clones mentioned above, were added to the test and control microplates. Both microplates were incubated at room temperature for 2 hours and washed three times with PBS containing 0.05% Tween. HRP-conjugated anti-M13 antibodies, diluted in PBS containing 0.05% Tween and 2% non-fat milk, were then added to both microplates. The plates were incubated at room temperature for 1 hour, washed three times with PBS containing 0.05% Tween, and HRP substrates were added to the microplates. The plates were then incubated at room temperature until a blue color

was developed. $OD_{450}$ and $OD_{650}$ values of each well were determined using an ELISA reader.

71 phage clones were found positive (HSV/control> 8) in the ELISA screening assay described above. cDNAs encoding the scFv expressed in these clones were amplified and their nucleotide sequences were determined. One of the positive phage clones expresses scFv E317 (SEQ ID NO:5) and another expresses scFv E425 (SEQ ID NO:6). Phage clone expresses scFv Y571 was also isolated.

EXAMPLE 2: Preparation of scFv E317, E425 and Y571

The cDNAs encoding scFv E317, E425, and Y571 were cloned into pET27b(+) expression vector and introduced into E. coli for expression. An E. coli clone carrying the cDNA encoding scFv E317, scFv E425, or scFv Y571 was incubated overnight at 37° C in Luria-Bertani (LB) medium supplemented with kanamycin. 70 ml of the overnight culture were inoculated into a fresh LB/kanamycin medium and cultured for 2 hours at 37° C. Isopropyl (β-D-1-thiogalactopyranoside (IPTG) was then added to the culture to a final concentration of 1 mM and the culture was further incubated at 30° C for 5 hours. E. coli cells were harvested via centrifugation, resuspended in Buffer A (50mM sodium phosphate, 1M sodium chloride, PH8.0), lysated by a microfludizer, and centrifuged again at 14,000 rpm for 20 minutes at 4° C to obtain a supernatant containing scFv E317, scFv E425, or scFv Y571, which is fused with a His-tag. The His-scFv fusion proteins were purified via affinity column chromatography following conventional procedures. The purified proteins were examined by polyacrylamide gel electrophoresis for purity and quantity.

The antigen-specificity of scFv E317 and scFv E425 was examined by an immunoprecipitation assay described below. 293T cells (2 X $10^5$) were transfected with 4 μg pLPCX-KOS-gD for expression of HSV glycoprotein D. After being cultured under suitable conditions for 48 hours, the transfected cells were harvested and lyzed using a lysis buffer containing PBS, 1% NP40, ImM PMSF, and protease inhibitors. 2 μg His-scFv E317 or His-scFv E425 was incubated with His-tag beads for one hour. After being washed with a phosphate buffer, the His-tag beads were collected and incubated with 10 μl of the cell lysate for one hour. The beads were then washed for several times with RIPA buffer containing 5mM EDTA and 1% Sodium Deoxycholate; proteins attached to the His-tag beads were eluted and subjected to western-blot analysis, using a commercially available anti-HSV glycoprotein D antibody (1:20000). Results thus obtained indicate that both scFv-317 and scFv-425 bound to HSV glycoprotein D. Using similar experiment, scFv Y571 was shown to bind to HSV glycoprotein D.

EXAMPLE 3: Inhibition of HSV Reproduction with scFv E317 and scFv E425

Plaque reduction assay was employed to examine the ability of scFv E317 and scFv E425 for inhibiting reproduction of HSV-1 and HSV-2. Briefly, 1 x $10^5$ Vero cells were seeded in each well of a 12-well plate and cultured in MEM medium at 37° C overnight. The medium was then replaced with a mixture (1 ml/well) containing IX PBS, MEM (FBS free), HSV-1 KOS, HSV-1 00410 (a clinical strain), HSV-2 186, or HSV-2 00040 (a clinical strain) (5 x $10^2$ pfu/ml for each virus strain), and one of the antibodies scFv E317 and scFv E425. Before placing in the plate, the mixture was pre-cultured at 37° C for one hour. An scFv E102 was used as a negative control. The Vero cells were incubated with the mixture at 37° C for 2 hr and the mixture was then removed. After being washed once with IX PBS, the plate containing the Vero cells was added with MEM containing 0.4% agarose and 10% FBS. After the agarose was solidified, the plate was placed in a 37° C incubator for 7 days. During this period, 500 μl MEM containing 10% FBS were added to each well in the plate. At day 8, the medium was removed from each well in the plate, and 500 μl IX PBS/ methanol (1:1 by volume) was then added. Five minutes later, the IX PBS/methanol was replaced with 500 μl 100% methanol to fix the cells contained in the wells. After another 5 min, the methanol was removed and the cells were stained with 250 μl crystal violet for 10 min. The crystal violet was then washed with water from the plate and the plate was air dried. The numbers of plaques contained in each well in the plate were then counted under a microscope.

The results obtained from the study describe above indicate that scFv E317, E425, and Y571 significantly inhibited plaque formation in Vero cells infected with either HSV-1 or HSV-2. The inhibitory effect of scFv E317, scFv E425, and scFv Y571 is summarized in Tables 1-3 below.

Table 1. Inhibitory Effect of scFv E317 on HSV-1 and HSV-2

| | scFv E317 | | | |
|---|---|---|---|---|
| | HSV-1 KOS | HSV-1 00410* | HSV-2 186 | HSV-200040* |
| $IC_{50}$ | 5.65 nM | 4.5 nM | 3.6 nM | 1.39 nM |
| (*: Clinical strains) | | | | |

Table 2. Inhibitory Effect of scFv E425 on HSV-1 and HSV-2

| | scFv E425 | | | |
|---|---|---|---|---|
| | HSV-1 KOS | HSV-1 00410* | HSV-2 186 | HSV-200040* |
| IC$_{50}$ | 2.2 nM | 1.8 nM | 4.5 nM | 1.16 nM |
| (*: Clinical strains) | | | | |

Table 3. Inhibitory Effect of scFv Y571 on HSV-1 and HSV-2

| | scFv Y571 | |
|---|---|---|
| | HSV-1 KOS | HSV-2 186 |
| IC$_{50}$ | 2.39 nM | 2.12 nM |

In Tables 1-3, IC$_{50}$ refers to the concentration of scFv E317, scFv E425, or scFv Y571 required to reach 50% inhibition on plaque formation in Vero cells infected with HSV. The percentage of inhibition is calculated following the formula below:

$$\% \text{ inhibition} = 100 - (\text{plaque number in HSV-infected cells in the presence of antibody})/(\text{plaque number in HSV-infected cells in the absence of antibody})$$

Control antibody scFv E102 failed to suppress plaque formation in Vero cells infected with either HSV-1 or HSV-2.

EXAMPLE 4: Protection from HSV lethality *in vivo*

In vivo protection studies from HSV-1 by mAb E317 was performed by treating mice inoculated with HSV-1 with antibody mAb E317-103 through intraperitoneal injection, followed by examining the survival rates of the infected mice. In particular, the studies were performed to analyze the effects of mAb E317 dosages and the effects of different mAb E317 dosing schedule (multiple doses versus single dose) on protection from HSV-1. The mouse strain used for these studies was C.B-17 SCID mouse. mAb E317-103 used in these studies is a full length antibody comprising two heavy chains and two light chains. The heavy and light variable regions of mAb E317-103 are from antibody scFv E317, the heavy chain constant region is from human IgG1 heavy chain, and the light chain constant region is from human kappa light chain.

Effect of multiple dose administration of mAb E317-103 on protection from HSV-1: Seven-week old male mice were used in this experiment. HSV-1 (KOS) was used to inoculate mice through intraperitoneal administration at the titer of $5 \times 10^3$ PFU. The mice were inoculated with HSV-1 virus on Day 0 (D0). mAb E317-103 was administered to mice through intraperitoneal injection every five days, i.e., it was administered at 24 hours prior to the virus inoculation (Day (-1) or D(-1)), 4 days after inoculation (D4), 9 days after inoculation (D9), 14 days after inoculation (D14), and 19 days after inoculation (D19). The study was performed using different mAb E317-103 dosages: 1.5 milligram antibody per kilogram of mouse body weight (mpk), 5 mpk, or 15 mpk. Two control groups were used, one receiving only phosphate buffer (PBS) ("Sham/Vehicle") and the other receiving only HSV-1 virus ("Disease/Vehicle"). There were 4 animals in Sham/Vehicle control group. There were 5 animals in Disease/Vehicle group and in each of the groups where antibody was administered. The survival rates were monitored daily for seven weeks after mice were inoculated with HSV-1.

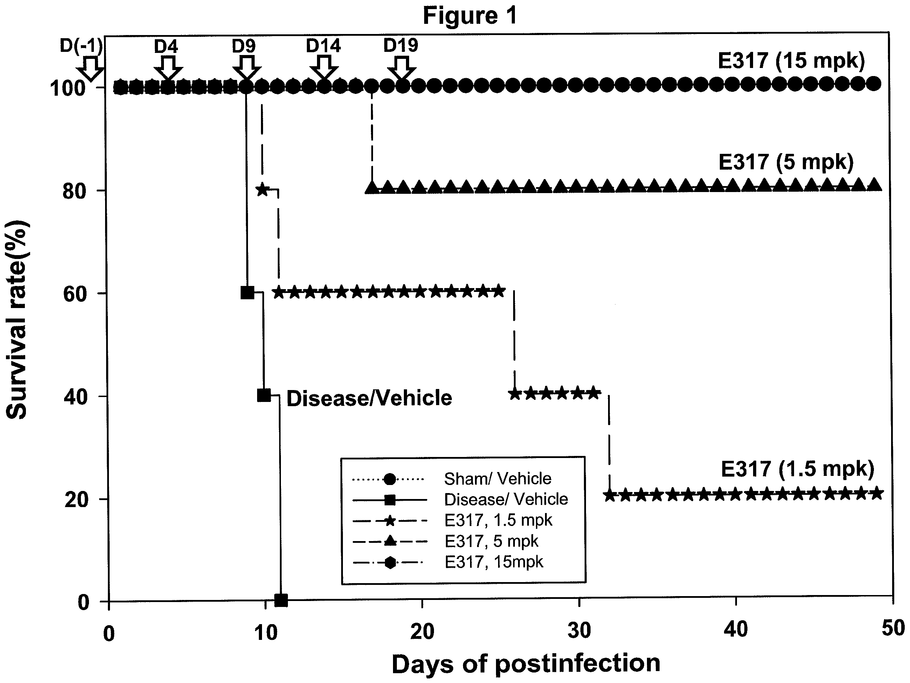

The results showed that administration of mAb E317-103 improved the survival of infected mice significantly (FIG. 1). All the mice in the Disease/Vehicle group died within 11 days of post-infection. The survival rates correlate positively with the mAb E317-103 dosage administered, with 100% survival rate for the mice receiving 15 mpk of the antibody, 80% survival rate for the mice receiving 5 mpk of the antibody, and 20% survival rate for the mice receiving 1.5 mpk of the antibody measured 7 weeks of post-infection.

Effect of single dose administration of mAb E317-103 on protection from HSV-1: Six-week old male mice were used in this study. HSV-1 (KOS) was used to inoculate mice through intraperitoneal administration at the titer of $5 \times 10^3$ PFU. The mice were inoculated with HSV-1 virus on Day 0 (DO). A single dose of mAb E317-103 was administered to mice at 24 hours prior to the virus inoculation (D(-1)). Different mAb E317-103 dosages were used: 1.5 mpk, 5 mpk, or 15 mpk. Two control groups were used, one receiving only PBS ("Sham/Vehicle") and the other receiving only HSV-1 virus ("Disease/Vehicle"). There were 5 animals in the Sham/Vehicle control group. There were 10 animals in the Disease/Vehicle control group and in each of the groups where antibody was administered. The survival rates were monitored daily for seven weeks after mice were inoculated with HSV-1.

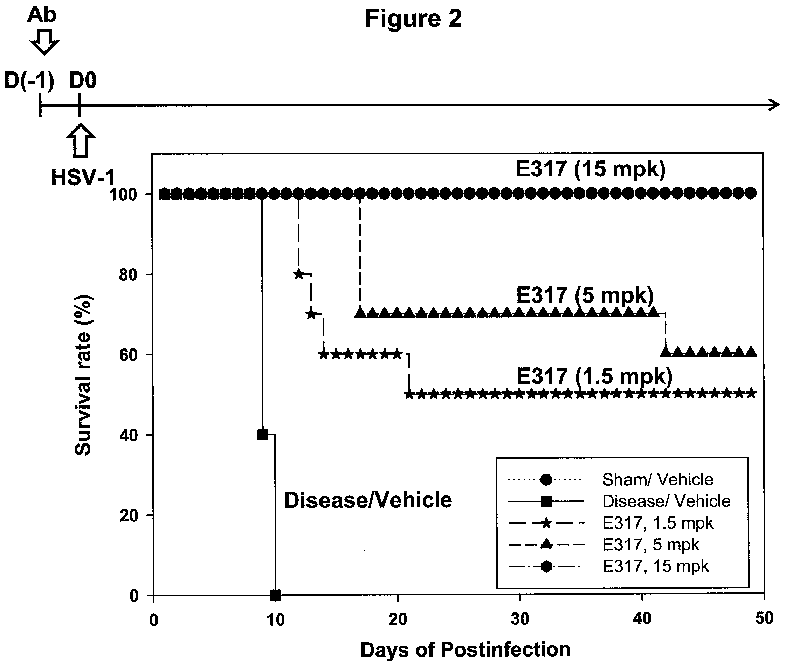

The results showed that, a single dose of mAb E317 was sufficient to provide protection from HSV-1 (FIG. 2). All the mice in the Disease/Vehicle group died within 10 days of post-infection. The survival rates correlate positively with the dosing of mAb E317, with 100% survival rate for the mice receiving 15 mpk of the antibody, 60% survival rate for the mice receiving 5 mpk of the antibody, and 50% survival rate for the mice receiving 1.5 mpk of the antibody about 7 weeks of post-infection. Therefore, a single dose administration of mAb E317 is effective in providing protection from HSV-1.

Effect of timing of mAb E317-103 administration on protection from HSV: Study was performed to analyze whether antibody was needed prior to virus challenge in order to be protective, or if the antibody could be administered on the same day or even after infection is established. Six-week old male mice were used in this study. HSV-1 (KOS) was used to inoculate mice through intraperitoneal administration at the titer of $5 \times 10^3$ PFU. The day mice were inoculated with HSV-1 virus was considered Day 0 (D0). mAb E317-103 was administered to mice at a single dosage of 15 mpk at 24 hours prior to the virus inoculation (D(-1)), on the same day of the virus inoculation (D0), or 24 hours after the virus inoculation (D1). For D0, virus was injected first and the antibody was injected right after; and the interval between the virus injection and the antibody injection was about 1 min. Two control groups were used, one receiving only PBS ("Sham/Vehicle") and the other receiving only HSV-1 virus ("Disease/Vehicle"). There were 5 animals in the Sham/Vehicle control group. There were 10 animals in the Disease/Vehicle control group and in each of the groups where antibody was administered. The survival rates were monitored daily for at least nine weeks after mice were inoculated with HSV-1.

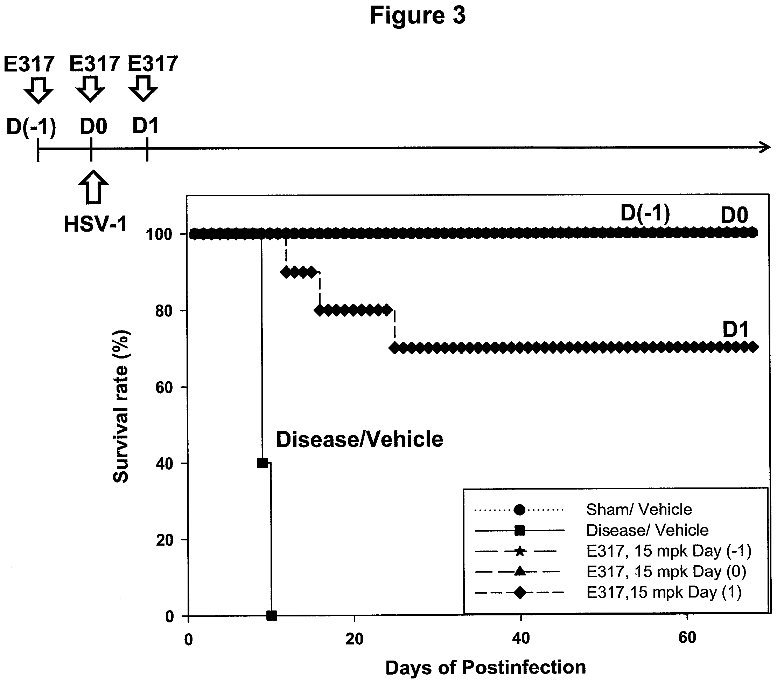

The results showed that administering mAb E317-103 at 24 hours prior to virus inoculation (D(-1)) and on the same day of inoculation (DO) provided complete protection (100 % survival rate for about nine weeks of post-infection) while administering mAb E317-103 at 24 hours post virus inoculation (D1) provided strong protection (70 % survival rate for about nine weeks of post-infection) (FIG. 3). Therefore, mAb 317-103 was protective when it was administered prior to infection or post-infection.

EXAMPLE 5: Characterization of the glycoprotein D epitope recognized by mAb E317 and mAb E425

Binding of mAb E317 and E425 to the gD is conformation dependent: The immunoprecipitation assay was performed to determine whether the glycoprotein ("gD") epitope by mAb E317 or E425 is conformation dependent. The assay was performed using existing methods known to one skilled in the art. Briefly, 293T cells were transfected with HSV-1 gD recombinant DNA (from KOS strain) expression construct and cells were harvested after 48 hours. The expression construction was generated by subcloning the cDNA encoding gD into pLPCX (Clontech) digested with XhoI and NotI sites inframe. Cells were lysed in buffer containing PBS, 1 % NP40, protease inhibitor, and ImM PMSF on ice for 10 minutes. Cell lysates were collected after centrifugation and incubated with HIS-tagged scFv E317 or E425 and HIS-select Nickel Affinity Gel beads (Sigma) in PBS at 4°C. Beads were collected and washed with RIPA buffer (50 mM Tris HCl pH7.4, 150 mM NaCl, 2 mM EDTA, 1% NP-40, 0.1% SDS) six times. The washed beads were analyzed by 12 % SDS-PAGE gel or Western Blot using mAb E317 or mAb E425. For the cell lysates immunoprecipitated by mAb E317, gD was shown on SDS-PAGE gel when the proteins on the gel was stained but not on the Western blot using mAb E317 or E425 for immunoblotting. That indicates that the mAb E317 or E425 was not able to bind denatured gD on the Western blot, and the gD epitope recognized by mAb E317 or E425 is not a linear epitope but is conformation dependent.

Mapping the gD epitope recognized by mAb E317 or E425: The gD epitope recognized by mAb E317 was mapped by examining the binding between mutated gD and mAb E317 or E425 through immunoprecipitation assays. The immunoprecipitation assay was performed using existing methods known to one skilled in the art. Briefly, 293T cells were transfected with HSV-1 gD recombinant DNA (from KOS strain) expression construct and cells were harvested after 48 hours. Cells were lysed in buffer containing PBS, 1 % NP40, protease inhibitor, and ImM PMSF on ice for 10 minutes. Cell lysates were collected after centrifugation and incubated with HIS-tagged scFv E317 and HIS-select Nickel Affinity Gel beads in PBS at 4°C. Beads were collected and washed with RIPA buffer six times. The washed beads were analyzed by performing Western Blot using a commercial anti-HSV gD monoclonal antibody H170 (ViruSys, Cat. No. PI 103) as the blotting antibody

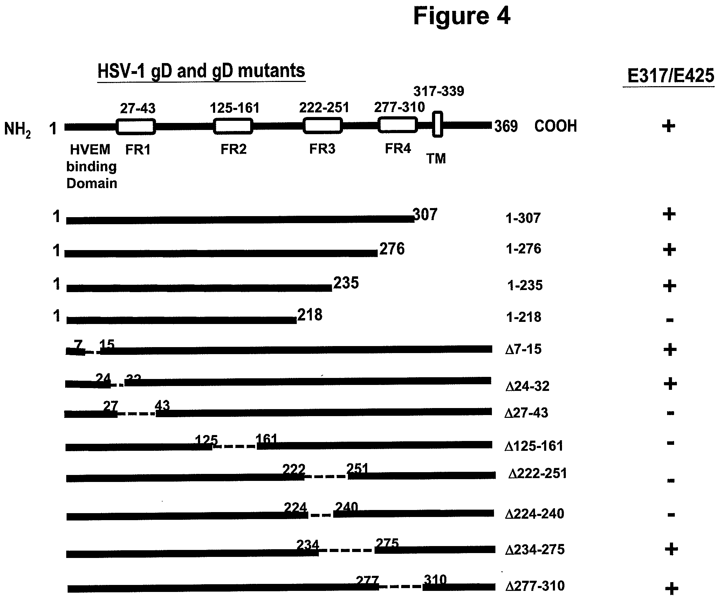

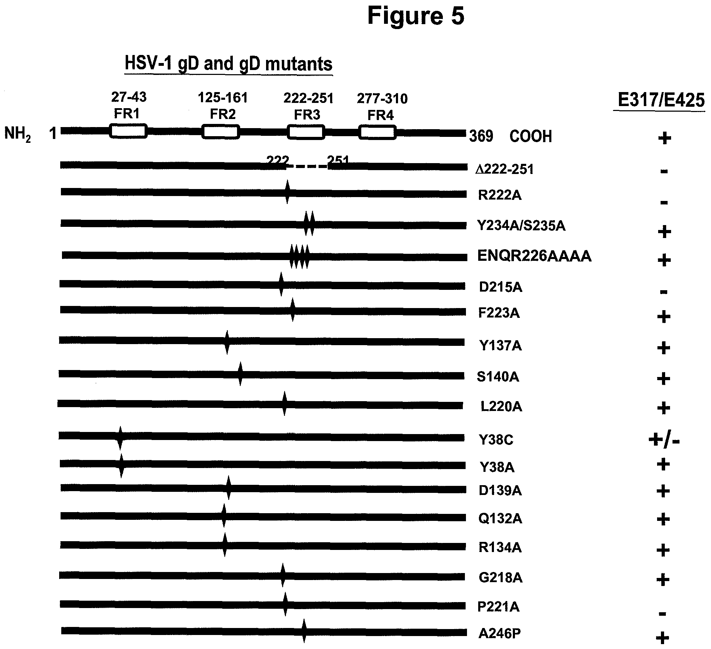

HSV-I gD mutants were generated by methods known to one skilled in the art. Study using truncated gD mutants showed that several regions in gD are important for antibody recognition (FIG. 4). Amino acids 218-235 seem to be important for antibody recognition since binding is shown for gD 1-235 but not for gD 1-218 or gD having deletion of amino acids 224-240. Amino acids 32-43 seem to be important for antibody recognition since binding is shown for gD having deletion of amino acids 24-32 but not for gD having deletion of amino acids 27-43. Deletion of amino acids 125-161 disrupts antibody recognition. Without wishing to be bound by theory, the deletion of amino acids 125-161 probably disrupts the protein conformation since the deletion disrupts a disulfide bond. Further mapping of the epitope was performed by using gD mutants generated through amino acid substitutions (for example, amino acid substitution of non-alanine amino acid with alanine). Several amino acids were found to be critical for antibody recognition, including Y38, D215, P221, and R222, since substitutions of these amino acids disrupted the recognition by mAb E317 or E425 (FIG. 5).

Therefore, the epitope recognized by mAb E317 or E425 is not a linear epitope and is conformation dependent, and amino acids Y38, D215, P221 and R222 are important for the binding of mAb E317 and E425 to the gD.

**Claims**

1.  An isolated human antibody that specifically binds to a herpes simplex virus (HSV) glycoprotein D, wherein the antibody comprises:

    (i) a heavy chain variable region (VH) comprising the three complementarity determining regions (CDRs) of the VH of scFv E317, as shown in SEQ ID NO:1, and a light chain variable region (VL) comprising the three CDRs of the VL of scFv E317, as shown in SEQ ID NO:2;
    (ii) a VH comprising the three CDRs of the VH of scFv E425, as shown in SEQ ID NO:3, and a VL comprising the three CDRs of the VL of scFv E425, as shown in SEQ ID NO:4; or
    (iii) a VH comprising the three CDRs from SEQ ID NO:41 and a VL comprising three CDRs from SEQ ID NO: 42.

2.  The antibody of claim 1, wherein the antibody comprises:

    (i) the VH and VL according to claim 1 (i) and wherein the VH comprises amino acids 3-124 of SEQ ID NO: 1 and the VL comprises amino acids 1-108 of SEQ ID NO: 2;
    (ii) the VH and VL according to claim 1 (ii) and wherein the VH comprises amino acids 3-124 of SEQ ID NO: 3 and the VL comprises amino acids 1-108 of SEQ ID NO: 4; or
    (iii) the VH and VL according to claim 1 (iii) and wherein the VH comprises the amino acid sequence shown in SEQ ID NO: 41 and the VL comprises the amino acid sequence shown in SEQ ID NO: 42.

3.  The antibody of claim 1, wherein the antibody comprises:

    (i) the VH and VL according to claim 1 (i) and wherein the antibody comprises the amino acid sequence shown in SEQ ID NO: 5;
    (ii) the VH and VL according to claim 1 (ii) and wherein the antibody comprises the amino acid sequence shown in SEQ ID NO: 6; or
    (iii) the VH and VL according to claim 1 (iii) and wherein the antibody comprises the amino acid sequence shown in SEQ ID NO: 43.

4.  An isolated nucleic acid comprising a nucleotide sequence encoding the antibody according to any one of the preceding claims.

5.  A vector comprising the nucleic acid of claim 4.

6.  An isolated host cell comprising the nucleic acid of claim 4.

7.  A method of producing the antibody of any one of claims 1 to 3, comprising culturing a host cell that produces the antibody, and recovering the antibody from the cell culture.

8.  A pharmaceutical composition comprising the antibody of any one of claims 1 to 3, and a pharmaceutically acceptable carrier.

9.  The antibody according to any one of claims 1 to 3 for use in a method of treating or preventing herpes simplex virus (HSV) infection, the method comprising administering to a subject in need thereof an effective amount of the antibody.

10. The antibody for use according to claim 9, wherein

    (a) the subject is at risk of HSV infection; or
    (b) the HSV infection is HSV- 1 or HSV-2 infection.

11. The antibody for use according to claim 9 or claim 10, wherein the method further comprises administering another anti-HSV drug.

12. A method of diagnosing HSV infection in a subject, comprising:

(a) contacting the antibody of any one of claims 1 to 3 with a biological sample from the subject and suspected of having HSV virus; and
(b) determining an antigen-antibody reaction; wherein detection of the antigen-antibody reaction indicates presence of HSV in the sample.


**Patentansprüche**

1. Isolierter menschlicher Antikörper, der spezifisch an ein Herpes-Simplex-Virus- (HSV-) Glykoprotein-D bindet, wobei der Antikörper Folgendes umfasst:

(i) eine variable Schwerkettenregion (VH), die die drei komplementaritätsbestimmenden Regionen (CDRs) der VH von scFv E317 wie in Seq.-ID Nr. 1 dargestellt umfasst, und eine variable Leichtkettenregion (VL), die die drei CDRs der VL von scFv E317 wie in Seq.-ID Nr. 2 dargestellt umfasst;
(ii) eine VH, die die drei CDRs der VH von scFv E425 wie in Seq.-ID Nr. 3 umfasst, und eine VL, die die drei CDRs der VL von scFv E425 wie in Seq.-ID Nr. 4 umfasst; oder
(iii) eine VH, die die drei CDRs von Seq.-ID Nr. 41 umfasst, und eine VL, die die drei CDRs von Seq.-ID Nr. 42 umfasst.

2. Antikörper nach Anspruch 1, wobei der Antikörper Folgendes umfasst:

(i) eine VH und eine VL nach Anspruch 1(i) und wobei die VH Aminosäuren 3-124 von Seq.-ID Nr. 1 und die VL Aminosäuren 1-108 von Seq.-ID Nr. 2 umfasst;
(ii) eine VH und eine VL nach Anspruch 1(ii) und wobei die VH Aminosäuren 3-124 von Seq.-ID Nr. 3 und die VL Aminosäuren 1-108 von Seq.-ID Nr. 4 umfasst; oder
(iii) eine VH und eine VL nach Anspruch 1 (iii) und wobei die VH die in Seq.-ID Nr. 41 dargestellte Aminosäuresequenz und die VL die in Seq.-ID Nr. 42 dargestellte Aminosäuresequenz umfasst.

3. Antikörper nach Anspruch 1, wobei der Antikörper Folgendes umfasst:

(i) eine VH und eine VL nach Anspruch 1(i) und wobei der Antikörper die in Seq.-ID Nr. 5 dargestellte Aminosäuresequenz umfasst;
(ii) eine VH und eine VL nach Anspruch 1(ii) und wobei der Antikörper die in Seq.-ID Nr. 6 dargestellte Aminosäuresequenz umfasst; oder
(iii) eine VH und eine VL nach Anspruch 1(iii) und wobei der Antikörper die in Seq.-ID Nr. 43 dargestellte Aminosäuresequenz umfasst.

4. Isolierte Nucleinsäure, umfassend eine Nucleotidsequenz, die für einen Antikörper nach einem der vorangegangenen Ansprüche kodiert.

5. Vektor, umfassend eine Nucleinsäure nach Anspruch 4.

6. Isolierte Wirtszelle, umfassend eine Nucleinsäure nach Anspruch 4.

7. Verfahren zur Erzeugung eines Antikörpers nach einem der Ansprüche 1 bis 3, umfassend das Kultivieren einer Wirtszelle, die den Antikörper erzeugt, und Gewinnen des Antikörpers aus der Zellkultur.

8. Pharmazeutische Zusammensetzung, umfassend einen Antikörper nach einem der Ansprüche 1 bis 3 und einen pharmazeutisch annehmbaren Träger.

9. Antikörper nach einem der Ansprüche 1 bis 3 zur Verwendung in einem Verfahren zur Behandlung oder Prävention einer Herpes-Simplex-Virus- (HSV-) Infektion, wobei das Verfahren das Verabreichen einer wirksamen Menge des Antikörpers an ein Individuum, das dies benötigt, umfasst.

**10.** Antikörper zur Verwendung nach Anspruch 9, wobei

(a) das Individuum von einer HSV-Infektion bedroht ist; oder
(b) die HSV-Infektion eine HSV-1- oder HSV-2-Infektion ist.

**11.** Antikörper zur Verwendung nach Anspruch 9 oder Anspruch 10, wobei das Verfahren ferner die Verabreichung eines weiteren Anti-HSV-Arzneimittels umfasst.

**12.** Verfahren zur Diagnose einer HSV-Infektion bei einem Individuum, umfassend:

(a) Kontaktieren eines Antikörpers nach einem der Ansprüche 1 bis 3 mit einer biologischen Probe von dem Individuum, bei dem Vorliegen des HSV-Virus vermutet wird; und
(b) Bestimmen einer Antigen-Antikörper-Reaktion, wobei Detektieren der Antigen-Antikörper-Reaktion das Vorliegen von HSV in der Probe anzeigt.

**Revendications**

**1.** Anticorps humain isolé qui se lie spécifiquement à la glycoprotéine D d'un virus herpès simplex (HSV), où l'anticorps comprend :

(i) une région variable de chaîne lourde (VH) comprenant les trois régions déterminant la complémentarité (CDR) de la VH du scFv E317, telle que montrée dans SEQ ID NO: 1, et une région variable de chaîne légère (VL) comprenant les trois CDR de la VL du scFv E317, telle que montrée dans SEQ ID NO: 2 ;
(ii) une VH comprenant les trois CDR de la VH du scFv E425, telle que montrée dans SEQ ID NO: 3, et une VL comprenant les trois CDR de la VL du scFv E425, telle que montrée dans SEQ ID NO: 4 ; ou
(iii) une VH comprenant les trois CDR de SEQ ID NO: 41 et une VL comprenant trois CDR de SEQ ID NO: 42.

**2.** Anticorps selon la revendication 1, où l'anticorps comprend :

(i) la VH et la VL selon la revendication 1 (i) et dans lequel la VH comprend les acides aminés 3-124 de SEQ ID NO: 1 et la VL comprend les acides aminés 1-108 de SEQ ID NO: 2 ;
(ii) la VH et la VL selon la revendication 1 (ii) et dans lequel la VH comprend les acides aminés 3-124 de SEQ ID NO: 3 et la VL comprend les acides aminés 1-108 de SEQ ID NO: 4 ; ou
(iii) la VH et la VL selon la revendication 1 (iii) et dans lequel la VH comprend la séquence d'acides aminés montrée dans SEQ ID NO: 41 et la VL comprend la séquence d'acides aminés montrée dans SEQ ID NO: 42.

**3.** Anticorps selon la revendication 1, où l'anticorps comprend :

(i) la VH et la VL selon la revendication 1 (i) et où l'anticorps comprend la séquence d'acides aminés montrée dans SEQ ID NO: 5 ;
(ii) la VH et la VL selon la revendication 1 (ii) et où l'anticorps comprend la séquence d'acides aminés montrée dans SEQ ID NO: 6 ; ou
(iii) la VH et la VL selon la revendication 1 (iii) et où l'anticorps comprend la séquence d'acides aminés montrée dans SEQ ID NO: 43.

**4.** Acide nucléique isolé comprenant une séquence de nucléotides codant pour l'anticorps selon l'une quelconque des revendications précédentes.

**5.** Vecteur comprenant l'acide nucléique selon la revendication 4.

**6.** Cellule hôte isolée comprenant l'acide nucléique selon la revendication 4.

**7.** Procédé de production de l'anticorps selon l'une quelconque des revendications 1 à 3, comprenant la mise en culture d'une cellule hôte qui produit l'anticorps, et la récupération de l'anticorps à partir de la culture cellulaire.

**8.** Composition pharmaceutique comprenant l'anticorps selon l'une quelconque des revendications 1 à 3, et un véhicule pharmaceutiquement acceptable.

**9.** Anticorps selon l'une quelconque des revendications 1 à 3 destiné à être utilisé dans un procédé de traitement ou de prévention d'une infection par le virus herpès simplex (HSV), le procédé comprenant l'administration, à un sujet en ayant besoin, d'une quantité efficace de l'anticorps.

**10.** Anticorps destiné à être utilisé selon la revendication 9, dans lequel :

(a) le sujet est exposé à un risque d'infection par le HSV ; ou
(b) l'infection par le HSV est une infection par le HSV-1 ou le HSV-2.

**11.** Anticorps destiné à être utilisé selon la revendication 9 ou la revendication 10, où le procédé comprend en outre l'administration d'un autre médicament anti-HSV.

**12.** Procédé de diagnostic d'une infection par le HSV chez un sujet, comprenant :

(a) la mise en contact de l'anticorps selon l'une quelconque des revendications 1 à 3 avec un échantillon biologique du sujet et suspecté de contenir un virus HSV ; et
(b) la détermination d'une réaction antigène-anticorps ; où la détection de la réaction antigène-anticorps indique la présence du HSV dans l'échantillon.

Figure 1

Figure 2

EP 2 379 590 B1

Figure 3

# Figure 4

## Figure 5

HSV-1 gD and gD mutants

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005011580 A2
- US 5108921 A
- US 5354844 A
- US 5416016 A
- US 55275285 B

### Non-patent literature cited in the description

- **CONNOLLY et al.** *J Virol.,* 2005, vol. 79 (2), 1282-95
- **RESKE et al.** *Rev. Med. Virol.,* 2007, vol. 17, 205-215
- **KARLIN ; ALTSCHUL.** *Proc, Natl. Acad. Sci. USA,* 1990, vol. 87, 2264-2268
- **KARLIN ; ALTSCHUL.** *Proc, Natl. Acad. Sci. USA,* 1993, 5873-5877
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **KABAT et al.** Sequences of Proteins of Immunological Interest. Public Health Service, National Institutes of Health, 1991
- **CHOTHIA ; LESK.** *J. Mol. Biol.,* 1987, vol. 196, 901-917
- **KASHMIRI et al.** *Methods,* 2005, vol. 36, 25-34
- **CLACKSON et al.** *Nature,* 1991, vol. 352, 624-628